# EUROPEAN PATENT APPLICATION

(11) **EP 4 732 853 A1**
(43) Date of publication of application: **29.04.2026**
(21) Application number: 24831962.6
(22) Date of filing: 26.06.2024
(51) Int. Cl.: A61L 2/20

(54) **STERILIZATION METHOD AND STERILIZATION DEVICE**

(30) Priority: 26.06.2023 JP 2023104201
(71) Applicant: The University of Osaka, Osaka 565-0871 (JP)
(72) Inventor: KITANO, Katsuhisa, Suita-shi, Osaka 565-0871 (JP); IKAWA, Satoshi, Izumi-shi, Osaka 594-1157 (JP)
(74) Representative: Plasseraud IP
(86) International application number: PCT/JP2024/023075
(87) International publication number: WO 2025/005102

(57) **Abstract**

A sterilization device (1) includes a first container (12) accommodating a target (A01), a first decompression section (14) that reduces a pressure in the first container, and a supply section (15) that supplies a peroxynitric acid gas to the target that is placed under a reduced pressure condition by the first decompression section. The sterilization device can sterilize a deep part of the target.

## Description

### Technical Field

The present invention relates to a sterilization method and a sterilization device.

### Background Art

A sterilization technology is one of basic technologies that support modern life. In the present specification, "sterilization" refers to reducing the number of living microorganisms. The sterilization process is intended for a wide range of targets, such as medical instruments, food containers, and food.

As an example of the background art, a sterilization method using a peroxynitric acid solution, developed by the inventors of the present invention is cited (see, for example, Patent Literature 1). According to this sterilization method, a peroxynitric acid solution is applied to a sterilization target.

### Citation List

### [Patent Literature]

[Patent Literature 1]
Japanese Patent No. 6087029

### Summary of Invention

### Technical Problem

However, for some targets of the sterilization process, it is difficult for the peroxynitric acid solution to reach deep parts thereof. Examples of this type of target include medical instruments (for example, syringes and tubes). Further, medical instruments are accommodated in a container and sterilized while being contained in the sterilization bag. The sterilization bag generally has a structure that makes it difficult for liquid, such as a peroxynitric acid solution, to pass therethrough. Therefore, with the background art, it may be difficult to sterilize a deep part, depending on the target.

It is an object of the present invention to provide a sterilization method and a sterilization device that enable sterilization of a deep part of a target.

### Solution to Problem

A sterilization method of the present invention includes supplying a peroxynitric acid gas to a target placed under a reduced pressure condition in a first container.

A sterilization device according to the present invention includes: a first container accommodating a target; a first decompression section that reduces a pressure in the first container; and a supply section that supplies a peroxynitric acid gas to the target that is placed under a reduced pressure condition by the first decompression section.

### Advantageous Effects of Invention

According to the present invention, it is possible to provide a sterilization method and a sterilization device that enable sterilization of a deep part of a target.

### Brief Description of Drawings

Fig. 1 is a view illustrating a configuration of a sterilization device in accordance with an embodiment.
Fig. 2 is a flowchart illustrating a sterilization process carried out by the sterilization device illustrated in Fig. 1.
Fig. 3 is a view illustrating a sterilization device in accordance with a first variation.
Fig. 4 is a flowchart illustrating a sterilization process carried out by the sterilization device illustrated in Fig. 3.
Fig. 5 is a view illustrating a sterilization device in accordance with a second variation.
Fig. 6 is a view illustrating a sterilization device in accordance with a third variation.
Fig. 7 is a perspective view illustrating a configuration of a discharge pipe.
Fig. 8 is a view schematically illustrating flow of gas in a sterilization device.

### Description of Embodiments

The following description will discuss a sterilization method and a sterilization device in accordance with an embodiment of the present disclosure with reference to the drawings. Note that, in the drawings, the same or corresponding parts are denoted by the same reference numerals, and redundant description thereof is omitted.

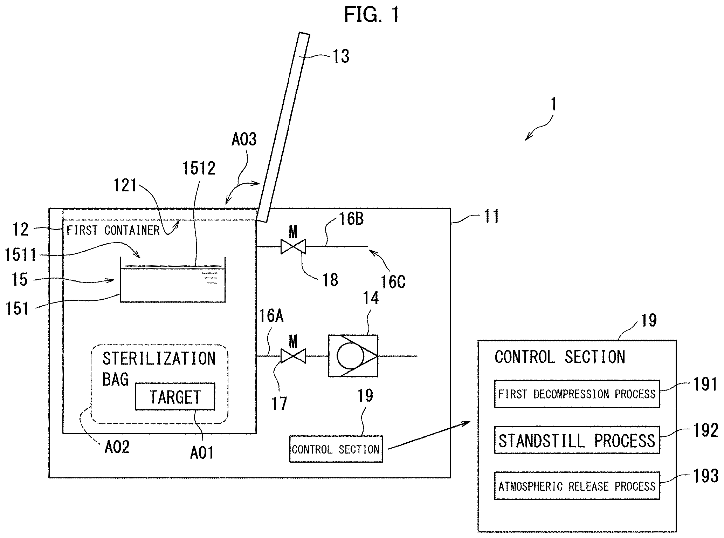

A sterilization method in accordance with the embodiment includes supplying a peroxynitric acid gas to a target AO1 (see Fig. 1) placed under a reduced pressure condition in a first container 12 (see Fig. 1). This enables sterilization of a deep part of the target A01. Specifically, the half-life of the peroxynitric acid gas is longer under a reduced pressure condition than otherwise. That is, under a reduced pressure condition, the lifetime of the peroxynitric acid gas is extended. This increases a distance by which the peroxynitric acid gas can be diffused in the first container 12 before the peroxynitric acid gas undergoes thermal deactivation. Therefore, even when the target A01 has a complex shape, a deep part of the target A01 is sterilized by the peroxynitric acid gas. Further, because the half-life of the peroxynitric acid gas becomes longer due to the pressure reduction, the concentration of the peroxynitric acid gas inside the first container 12 also becomes relatively high. This enhances the microbicidal activity exerted by the peroxynitric acid gas, and a strong sterilization effect is obtained in a short time.

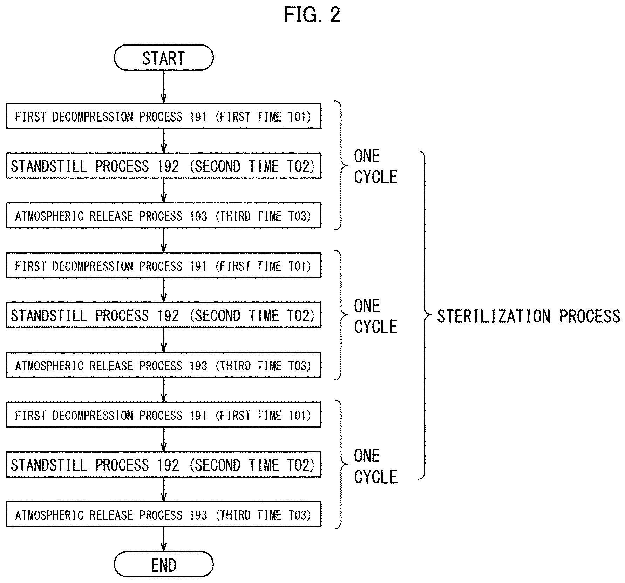

Fig. 1 is a view illustrating a sterilization device 1 for carrying out a sterilization method. Fig. 2 is a flowchart illustrating a sterilization process carried out by the sterilization device 1 illustrated in Fig. 1.

As illustrated in Fig. 1, the sterilization device 1 includes, as components thereof, a housing 11, the first container 12, a cover 13, a first decompression section 14, a supply section 15, flow paths 16A and 16B, an atmospheric release port 16C, a first valve 17, an atmospheric release valve 18, and a control section 19.

The housing 11 includes an outer casing and a frame of the sterilization device 1, and accommodates the components other than the cover 13.

The first container 12 is capable of accommodating the target AO1. The target A01 is to be subjected to the sterilization process carried out by the present sterilization device 1. Typically, the target A01 is a medical instrument. Some medical instruments have shapes which it is difficult for liquid, such as a peroxynitric acid solution, to enter. Examples of such medical instruments include catheters. Other examples of the medical instruments include syringes. Further, the medical instruments and the like are accommodated in the first container 12 while being contained in a sterilization bag A02. The sterilization bag A02 does not allow liquid, e.g., a peroxynitric acid solution to pass therethrough. Therefore, it is difficult to use the peroxynitric acid solution to sterilize the target A01 contained in the sterilization bag A02. Note that the target A01 is not limited to the medical instrument but also includes a wide variety of items, such as food containers, food, and seeds.

Hereinafter, the term "target A01" includes the meaning of the target A01 itself and the meaning of the target A01 contained in the sterilization bag A02.

Typically, the first container 12 is a pressure container. The pressure container is designed to be capable of storing a substance under a pressure different from the atmospheric pressure. The first container 12 has an opening 121 facing in a predetermined direction. That is, the first container 12 is provided with the opening 121.

The cover 13 is attached to the housing 11 so as to enable the opening 121 to be opened and closed (see arrow A03). The cover 13 seals the first container 12 while being closed. While the cover 13 is open, an operator carries out, through the opening 121, operations such as accommodating the target A01 into the first container 12 and putting a peroxynitric acid solution into a storage part 151 of the supply section 15.

The first decompression section 14 reduces the pressure inside the first container 12. Specifically, under the control of the control section 19, the first decompression section 14 discharges the gas inside the first container 12 closed by the cover 13 to the outside of the first container 12. Further, under the control of the control section 19, the first decompression section 14 maintains the reduced pressure condition inside the first container 12 closed by the cover 13. Typically, the first decompression section 14 is a vacuum pump.

The supply section 15 supplies a peroxynitric acid gas to the target A01 that is placed under a reduced pressure condition by the first decompression section 14. The peroxynitric acid gas has a relatively long half-life under a reduced pressure condition, and thus can sterilize a deep part of the target A01.

In the embodiment, the supply section 15 includes the storage part 151, which is a bottomed container. The storage part 151 is placed inside the first container 12. The storage part 151 has an opening 1511. The opening 1511 faces upward. The storage part 151 stores a peroxynitric acid solution. As a result, in the first container 12, the peroxynitric acid solution evaporates, thereby generating a peroxynitric acid gas directly above the opening 1511.

The flow path 16A connects the inside of the first container 12 and the first decompression section 14. The flow path 16A is a pipe made of metal or resin, and connects the inside of the first container 12 and the first decompression section 14 so as to allow a fluid to flow therebetween. The flow path 16A is one example of "first flow path" in the present invention.

The first valve 17 is a control valve provided in the flow path 16A. Under the control of the control section 19, the first valve 17 opens and closes the flow path 16A.

The flow path 16B has, at one end thereof, the atmospheric release port 16C. The atmospheric release port 16C is an opening opened to the atmospheric air. The flow path 16B is a pipe made of metal or resin. The other end of the flow path 16B is connected to the inside of the first container 12. The flow path 16B extends from the inside of the first container 12 to the atmospheric release port 16C. The flow path 16B allows a fluid to pass therethrough.

The atmospheric release valve 18 is a valve for releasing the pressure inside the first container 12 to the atmospheric air. This enables increase of the pressure inside the first container 12 with a simple configuration. Specifically, the atmospheric release valve 18 is a control valve that is provided in a flow path 181 and opens and closes the flow path 181 under the control of the control section 19.

The control section 19 includes a processor and a memory. The processor is a central processing unit or a microcomputer. The memory includes a read-only memory and a random access memory. The memory stores a computer program. The computer program specifies operations of the processor.

In the control section 19, the processor carries out the computer program. As a result, the control section 19 repeatedly carries out a first decompression process 191, a standstill process 192, and an atmospheric release process 193 in this order (see Fig. 2). The first decompression process 191 and the atmospheric release process 193 are one example of "decompression step" and one example of "atmospheric release step" in the present invention, respectively.

In the embodiment, the period from the start of one first decompression process 191, through one standstill process 192, to the end of one atmospheric release process 193 is defined as "one cycle". Fig. 2 illustrates a process containing three cycles as one example.

As a stage prior to the first decompression process 191, an operator accommodates the target A01 into the first container 12 after opening the cover 13. Further, the operator puts a peroxynitric acid solution into the storage part 151 according to need. Thereafter, the operator closes the cover 13 and causes the sterilization device 1 to carry out a sterilization process (see Fig. 2). As illustrated in Fig. 2, the sterilization process includes the first decompression process 191, the standstill process 192, and the atmospheric release process 193.

In Figs. 1 and 2, the first decompression process 191 involves so-called "vacuuming". Specifically, the control section 19 opens the first valve 17, closes the atmospheric release valve 18, and reduces the pressure inside the first container 12 with use of the first decompression section 14. Inside the first container 12, a peroxynitric acid gas is generated above the opening 1511 through evaporation. The first container 12 is filled with the peroxynitric acid gas, resulting in supply of the peroxynitric acid gas to the sterilization bag A02 through the supply section 15. The peroxynitric acid gas, which is in the form of gas, passes through the sterilization bag A02, enters the sterilization bag A02, and reaches the target A01.

Due to the reduced pressure condition inside the first container 12 created by the first decompression process 191, the half-life of the peroxynitric acid gas becomes longer. This increases a distance by which the peroxynitric acid gas can be diffused in the first container 12 before the peroxynitric acid gas undergoes thermal deactivation. As a result, it becomes easier for the peroxynitric acid gas to reach a deep part of the target A01. As such, the sterilization device 1 enables sterilization of a deep part of the target A01. Further, as the half-life becomes longer, the concentration of the peroxynitric acid gas inside the first container 12 becomes relatively high. This enhances microbicidal activity by the peroxynitric acid gas, thus providing a strong sterilization effect in a short time.

Note that, in order to suppress evaporation of water from the peroxynitric acid solution by the first decompression process 191, it is preferable that the pressure inside the first container 12 be set to a value higher than the saturated vapor pressure of water (for example, approximately 0.02 atm at 20°C).

The control section 19 transitions to the standstill process 192 on condition that a first time TO1 (that is, vacuuming time) has elapsed from the start of the first decompression process 191. Note that this condition may be that a degree of vacuum inside the first container 12 reaches a predetermined threshold. In the standstill process 192, while maintaining the atmospheric release valve 18 in a closed state, the control section 19 closes the first valve 17 and stops the first decompression section 14. The control section 19 transitions to the atmospheric release process 193 on condition that a second time T02 (that is, a standstill time) has elapsed from the start of the standstill process 192. Note that the standstill process may not be carried out. That is, the sterilization process may transition from the first decompression process 191 directly to the atmospheric release process 193. In this case, the second time T02 is zero.

The atmospheric release process 193 involves so-called "vacuum break". Specifically, the control section 19 opens the atmospheric release valve 18 to open the inside of the first container 12 to the atmospheric air. Preferably, the control section 19 opens the atmospheric release valve 18 with the first valve 17 closed and with operation of the first decompression section 14 stopped. As a result, the atmospheric air is introduced into the first container 12, resulting in pressure change inside the first container 12, and the peroxynitric acid gas above the opening 1511 moves in the first container 12. Accordingly, above the opening 1511, gas in which the partial pressure of the peroxynitric acid gas is relatively low is supplied. This promotes evaporation of the peroxynitric acid solution in the storage part 151, and new peroxynitric acid gas is generated above the opening 1511. Therefore, the sterilization capability of the sterilization device 1 is maintained for a long time.

The first decompression process 191 increases the half-life of the peroxynitric acid gas. Diffusion of such peroxynitric acid gas inside the first container 12 makes it possible to sterilize a deep part of the target A01. However, some targets A01 have a portion that is difficult to sterilize, such as a "thin tube with a sealed tip end". To achieve sterilization of such targets A01, in the embodiment, the first decompression process 191 and the atmospheric release process 193 are repeated for a plurality of cycles, and as a result, a fluidic flow is created inside the first container 12.

However, in the case of peroxynitric acid gas, when by the atmospheric release process 193, the first container 12 is opened to the atmospheric air, the half-life of the peroxynitric acid gas decreases with the increase in pressure. As a result, the sterilization capability of the peroxynitric acid gas is lost in a short time. In the embodiment, in order to maintain the sterilization capability of the peroxynitric acid gas for a long time, the sterilization device 1 periodically repeats the first decompression process 191, so that the first container 12 is filled with the peroxynitric acid gas under a reduced pressure condition. In evacuation by a general vacuum pump, the time required for the atmospheric release process 193 is comparable to the half-life of the peroxynitric acid gas. Therefore, it is preferable to repeatedly carry out the first decompression process 191, the standstill process 192, and the atmospheric release process 193 again, immediately after the outside air has been introduced into the first container 12 by the atmospheric release process 193.

The peroxynitric acid gas has a property that its sterilization activity is likely to be deactivated due to thermal deactivation under an increased pressure condition. However, in the embodiment, by repeating the first decompression process 191 and the atmospheric release process 193 for a plurality of cycles, the peroxynitric acid gas having the above-described property effectively reaches a deep part of the target A01.

In the atmospheric release process 193, a jet of air is preferably supplied from the flow path 16B so that the jet of air strikes the liquid surface of the peroxynitric acid solution in the storage part 151. This enables the air introduced into the first container 12 to sufficiently contain the peroxynitric acid gas above the opening 1511 of the storage part 151. Diffusion of such mixed gas inside the first container 12 allows the first container 12 to be filled with the peroxynitric acid gas under a reduced pressure condition. As a result, the sterilization capability of the sterilization device 1 is enhanced.

The peroxynitric acid gas is generated by evaporation of the peroxynitric acid solution. Therefore, no salt is released from the peroxynitric acid solution into the first container 12. This prevents contamination of the target AO1.

Note that, although there are several methods for generating a peroxynitric acid solution, the peroxynitric acid solution is generally generated by mixing a nitrous acid solution and a hydrogen peroxide solution under an acidic condition. Since the nitrous acid solution can be made from a nitrite (e.g., sodium nitrite) and nitric acid, the peroxynitric acid solution is generated by dropping a nitrite solution into a solution in which a hydrogen peroxide solution and a nitric acid solution are mixed. In this case, the peroxynitric acid solution may contain a nitrate (e.g., sodium nitrate) as a salt.

It is preferable that, as illustrated in Fig. 1, an absorbent body 1512 prepared from paper or polymer fiber be additionally placed on the liquid surface of the peroxynitric acid solution in the storage part 151. The absorbent body 1512 is more preferably in sheet form. The absorbent body 1512 absorbs the peroxynitric acid solution by capillary action. This increases the area of the peroxynitric acid solution that is in direct contact with the gas above the opening 1511. As a result, evaporation of the peroxynitric acid solution is further promoted. Therefore, the absorbent body 1512 can also be regarded as an evaporation promoter.

The control section 19 transitions to the first decompression process 191 on condition that a third time T03 (i.e., break time) has elapsed from the start of the atmospheric release process 193. A fourth time T04, which is the total time of the second time T02 and the third time T03, is determined on the basis of the half-life of the peroxynitric acid gas. Therefore, the first decompression process 191 is repeated at a period based on the half-life of the peroxynitric acid gas. By repeating the first decompression process 191 at a period based on the half-life, the sterilization capability of the sterilization device 1 is maintained. Note that in a case where the first decompression process 191 is repeated at a period much longer than the half-life, the sterilization capability of the peroxynitric acid gas may be lost due to thermal deactivation. Such peroxynitric acid gas, even if it can reach a deep part of the target A01, cannot sterilize the deep part of the target A01.

Regarding a half-life of peroxynitric acid gas, the following reference literature discloses a formula.
[Reference literature] Richard A. Graham, Arthur M. Winer, and James N. Pitts Jr., The Journal of Chemical Physics "Pressure and temperature dependence of the unimolecular decomposition of HO2NO2" published in the United States, 1978, Vol. 68, p. 4505

According to the formula, for example, the peroxynitric acid gas has a half-life of approximately 97 seconds under conditions of 1 atmospheric pressure and 4.55°C (277.7 K). Further, the half-life is approximately 12 seconds under conditions of 1 atmospheric pressure and 24.85°C (298 K). Note that, under a reduced pressure condition, the half-life of the peroxynitric acid gas depends also on the pressure.

Generally, the half-life of the peroxynitric acid gas is shorter than the half-life of the peroxynitric acid solution. Therefore, if the fourth time is much longer than the half-life or the first time T01, the peroxynitric acid gas fails to reach a deep part of the target A01. Therefore, it is not preferable that the fourth time becomes excessively longer than the half-life or the first time T01.

The inventors conducted the following experiment in order to confirm the effect of the sterilization method and the sterilization device 1 in accordance with the embodiment.

The inventors prepared the following three types of tubes A, B, and C made of Teflon (registered trademark). Each of the tubes A to C was 1/8 inch (inner diameter: 1.59 mm, outer diameter: 3.17 mm). The lengths of the tubes A, B, and C were 100cm, 300cm, and 500cm, respectively.

The inventors connected a syringe to each of the tubes A to C and inserted a biological indicator (manufactured by MesaLabs, Inc., model number: HMV-091) into the syringe. The biological indicator (hereinafter, referred to as "BI") is used to evaluate the effect of the sterilization device. The BI has a structure in which a carrier to which bacterial spores are applied is wrapped with a non-woven fabric. Of droplets and gas, only gas can pass through the non-woven fabric. Therefore, if bacteria inside the BI are sterilized, it can be determined that the sterilization has been carried out by gas. For the non-woven fabric used for the sterilization bag and the BI, a material such as Tyvek (registered trademark), which has a good permeability to the peroxynitric acid gas, is suitable.

The inventors evaluated a sterilization capability of the sterilization device 1 with use of the biological indicators provided to the tubes A to C with the first time T01 (vacuuming time) set to one minute, the second time T02 (standstill time) set to one minute, and the third time T03 (break time) set to one minute. By the first decompression process 191, the pressure inside the first container 12 is reduced to 0.1 atm. Further, by the atmospheric release process 193, air at 0.9 atm was introduced into the first container 12. For the sterilization device 1, a process including the first decompression process 191, the standstill process 192, and the atmospheric release process 193 in this order was repeated for three cycles. As a result, all the biological indicators indicated less than 30 CFU (colony forming unit).

That is, the inventors confirmed that the sterilization device 1 successfully carried out sterilization for 9 minutes in total.

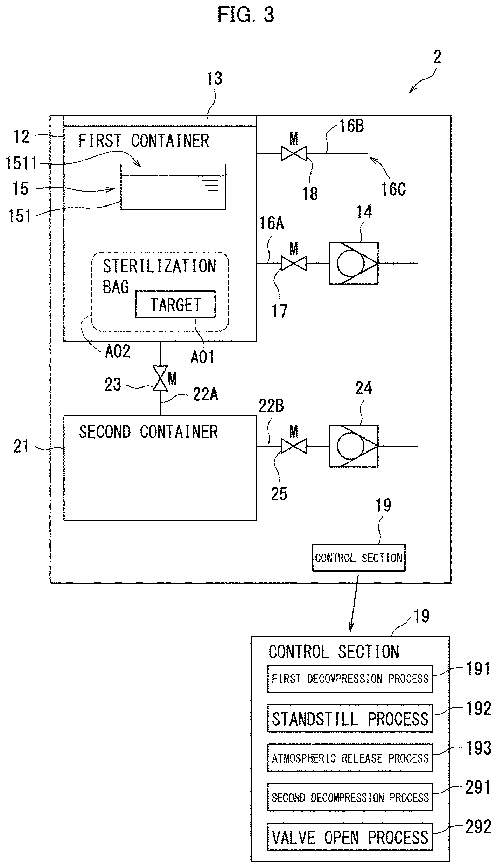

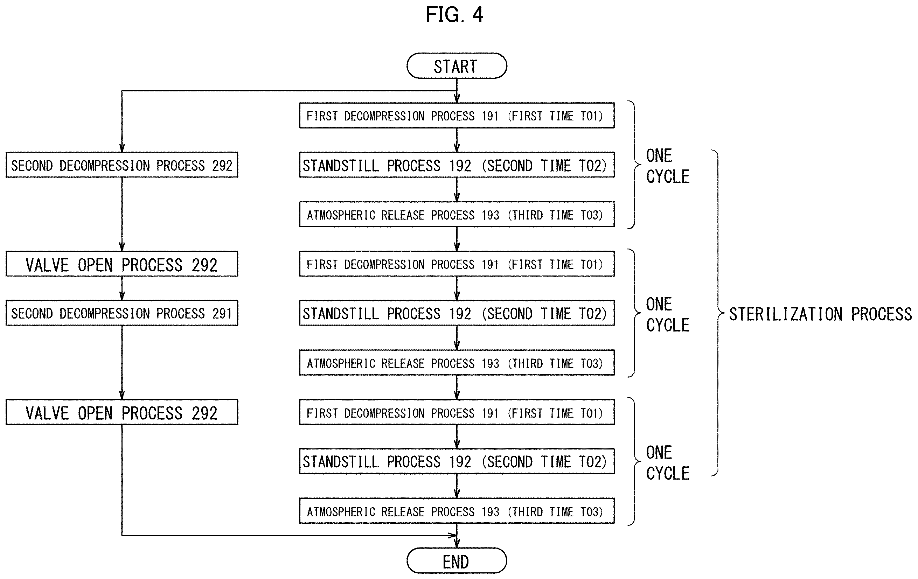

Fig. 3 is a view illustrating a configuration of a sterilization device 2 in accordance with a first variation. Fig. 4 is a flowchart illustrating a sterilization process carried out by the sterilization device 2 illustrated in Fig. 3.

As illustrated in Fig. 3, in comparison with the sterilization device 1 (see Fig. 1), the sterilization device 2 further includes a second container 21, flow paths 22A and 22B, a second valve 23, a second decompression section 24, and a third valve 25.

The second container 21 can be a pressure container with specifications identical to those of the first container 12.

The flow path 22A connects the inside of the first container 12 and the inside of the second container 21. The flow path 22A is a pipe made of metal or resin, and connects the inside of the first container 12 and the inside of the second container 21 so as to allow a fluid to flow between the two. The flow path 22A is one example of "second flow path" in the present invention.

The second valve 23 is a control valve provided in the flow path 22A. Under the control of the control section 19, the second valve 23 opens and closes the flow path 22A.

The second decompression section 24 reduces the pressure inside the second container 21. Specifically, under the control of the control section 19, the second decompression section 24 discharges the gas inside the second container 21 to the outside of the second container 21. Further, under the control of the control section 19, the second decompression section 24 maintains the reduced pressure condition inside the second container 21. Typically, the second decompression section 24 is a vacuum pump. Note that the second decompression section 24 may be the vacuum pump that constitutes the first decompression section 14.

The flow path 22B connects the inside of the second container 21 and the second decompression section 24. The flow path 22B is a pipe made of metal or resin, and connects the inside of the second container 21 and the second decompression section 24 so as to allow a fluid to flow therebetween. The flow path 22B is one example of "third flow path" in the present invention.

The third valve 25 is a control valve provided in the flow path 22B. Under the control of the control section 19, the third valve 25 opens and closes the flow path 22B.

As in the case of the embodiment, the operation at the stage prior to the first decompression process 191 is carried out by an operator. Thereafter, the sterilization process by the sterilization device 2 is started. As illustrated in Fig. 4, the sterilization process differs from the sterilization process illustrated in Fig. 2 in further involving a second decompression process 291 and a valve open process 292.

In Figs. 3 and 4, the second decompression process 291 involves so-called "vacuuming". Specifically, the second decompression process 291 is carried out in a timing different from the first decompression process 191. The second decompression process 291 is thus carried out in a time period when the standstill process 192 and the atmospheric release process 193 are carried out.

Specifically, in the second decompression process 291, the control section 19 opens the third valve 25 and reduces the pressure inside the second container 21 with use of the second decompression section 24. In the second decompression process 201, the second valve 23 is preferably closed.

The valve open process 292 is carried out by the control section 19 while the first decompression process 191 is being carried out. The control section 19 preferably carries out the valve open process 292 during a period from the start of the first decompression process 191 to the end of the first decompression process 191. In the valve open process 292, the control section 19 opens the second valve 23. As a result, in the first decompression process 191, the pressure inside the first container 12 is reduced relatively quickly.

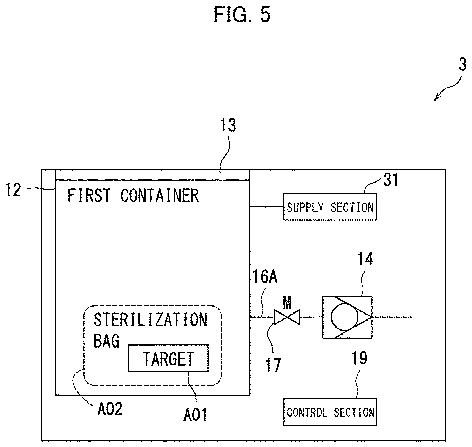

Fig. 5 is a view illustrating a sterilization device 3 in accordance with a second variation.

As illustrated in Fig. 5, the sterilization device 3 differs from the sterilization device 1 (see Fig. 1) in including a supply section 31 instead of the supply section 15, the flow path 16B, and the atmospheric release valve 18.

The supply section 31 is a bubbling device provided outside the first container 12. The supply section 31 supplies, into the first container 12, a peroxynitric acid gas obtained by bubbling the peroxynitric acid solution. The peroxynitric acid gas diffuses in the first container 12, and the first container 12 is filled with the peroxynitric acid gas under a reduced pressure condition. Specifically, the supply section 31 flows a carrier gas into a tank storing a peroxynitric acid solution, and supplies a mixed gas of the vaporized peroxynitric acid solution (that is, peroxynitric acid gas) and the carrier gas into the first container 12. That is, the mixed gas allows the outside air (that is, carrier gas) to be introduced into the first container 12. Further, by passing the outside air through the peroxynitric acid solution (bubbling the peroxynitric acid solution), a mixed gas containing a large amount of peroxynitric acid gas is introduced into the first container 12. This makes it possible for the air introduced into the first container 12 to sufficiently contain the peroxynitric acid gas. Diffusion of such mixed gas inside the first container 12 allows the first container 12 to be filled with the peroxynitric acid gas under a reduced pressure condition. As a result, the sterilization capability of the sterilization device 1 is enhanced.

The supply section 31 may control the flow of the carrier gas with use of, for example, an electromagnetic valve, or may control the flow of the carrier gas with use of, for example, a mass flow controller.

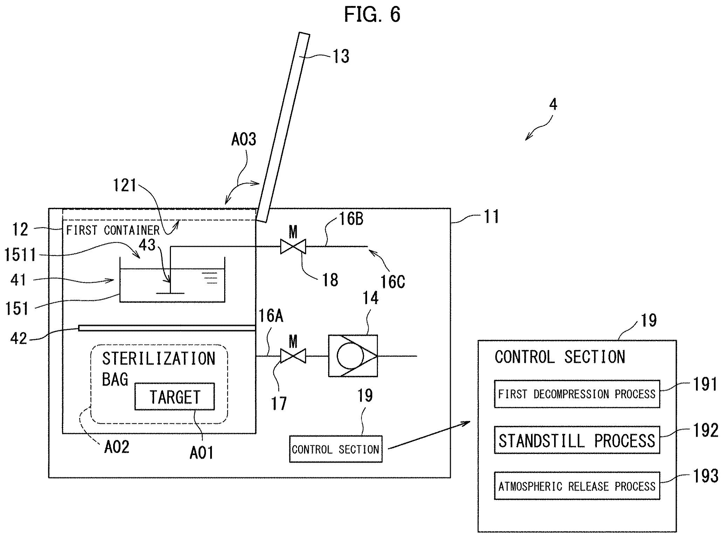

Fig. 6 is a view illustrating a sterilization device 4 in accordance with a third variation. In comparison with the sterilization device 1 (see Fig. 1), the sterilization device 4 includes a supply section 41 and a partition 42 instead of the supply section 15.

The partition 42 is a wall partitioning the inside of the first container 12. The partition 42 separates a space where the target AO1 is disposed from a space where the supply section 41 is disposed. The partition 42 is partially left open so as to allow the space where the target A01 is disposed and the space where the supply section 41 is disposed to communicate.

The supply section 41 has the storage part 151 storing a peroxynitric acid solution and a discharge pipe 43. The supply section 41 constitutes a bubbler.

The discharge pipe 43 has a base end connected to the flow path 16B. The discharge pipe 43 has a tip end disposed in the peroxynitric acid solution in the storage part 151. The discharge pipe 43 is a pipe for introducing air (carrier gas) into the peroxynitric acid solution to carry out bubbling. By supplying bubbles of air into the peroxynitric acid solution by bubbling, evaporation of the peroxynitric acid solution is further promoted, thus making it possible to provide a gas containing a large amount of peroxynitric acid gas.

Note that the container in which the supply section 41 is disposed and the container in which the target A01 is disposed may be separate vacuum vessels connected to each other by a flow path. For example, the supply section 31 in Fig. 5 is used as a bubbler.

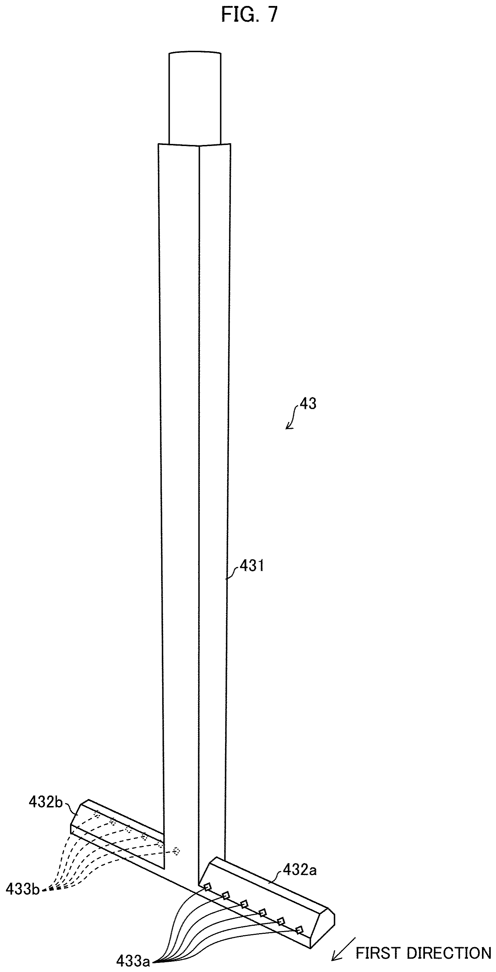

Fig. 7 is a perspective view illustrating a configuration of the discharge pipe 43. The discharge pipe 43 includes a trunk pipe 431, a first branch pipe 432a, and a second branch pipe 432b. The air from the flow path 16B is introduced into an upper part of the trunk pipe 431. The first branch pipe 432a is connected to the tip end (lower part) of the trunk pipe 431, and the second branch pipe 432b is connected on a side opposite to the first branch pipe 432a.

The first branch pipe 432a and the second branch pipe 432b extend laterally. The first branch pipe 432a has, on a side surface on one side (on a first direction side), a plurality of discharge ports 433a. The second branch pipe 432b has, on the side surface on the other side (on a side opposite to the first direction), a plurality of discharge ports 433b. Air is discharged from the plurality of discharge ports 433a and the plurality of discharge ports 433b. The first branch pipe 432a and the second branch pipe 432b have a cross-sectional shape like an airplane wing. For example, the first branch pipe 432a and the second branch pipe 432b have, on a first direction side and on a side opposite to the first direction, inclined surfaces facing obliquely upward.

The discharge pipe 43 discharges air from the plurality of discharge ports 433a and the plurality of discharge ports 433b in a lateral direction, an obliquely downward direction, or an obliquely upward direction. The bubbles of the discharged air thus create a lateral flow in the peroxynitric acid solution. Therefore, compared to a case where air is simply discharged downward or upward, the time for which the bubbles are present in the peroxynitric acid solution can be extended. This makes it possible to increase the concentration of the peroxynitric acid gas contained in the bubbles. Further, since bubbling is carried out under reduced pressure, the volume of the air introduced into the supply section 41 increases significantly. In order to suppress scattering of the peroxynitric acid solution and to pass a large volume of air through the peroxynitric acid solution, a configuration, such as the discharge pipe 43, that discharges air in a rotational direction is preferable.

Furthermore, the plurality of discharge ports 433a and the plurality of discharge ports 433b discharge air in directions opposite to each other. Therefore, the bubbles of the discharged air create a rotating spiral flow in the peroxynitric acid solution. This makes it possible to further extend the time for which the bubbles are present in the peroxynitric acid solution and to promote contact between the bubbles and the peroxynitric acid solution. Further, the first branch pipe 432a and the second branch pipe 432b, which have a cross-sectional shape like an airplane wing, have a structure that is unlikely to interrupt the spiral flow of the peroxynitric acid solution. For example, the trunk pipe 431 is disposed at the center of the storage part 151 in a plan view.

Note that the discharge pipe may have a configuration having only one of the first branch pipe and the second branch pipe. The first branch pipe and the second branch pipe may be each configured to have one discharge port. It is sufficient that the discharge pipe can create a flow that rotates in a plan view in the peroxynitric acid solution. The storage part 151 may have a cylindrical shape. The discharge ports of the discharge pipe may have a configuration that discharges air in a circumferential direction (rotational direction) in a position apart from the center of the storage part 151.

Since the space inside the first container 12 is divided by the partition 42, maintenance such as cleaning of splashes around the supply section 41 is facilitated.

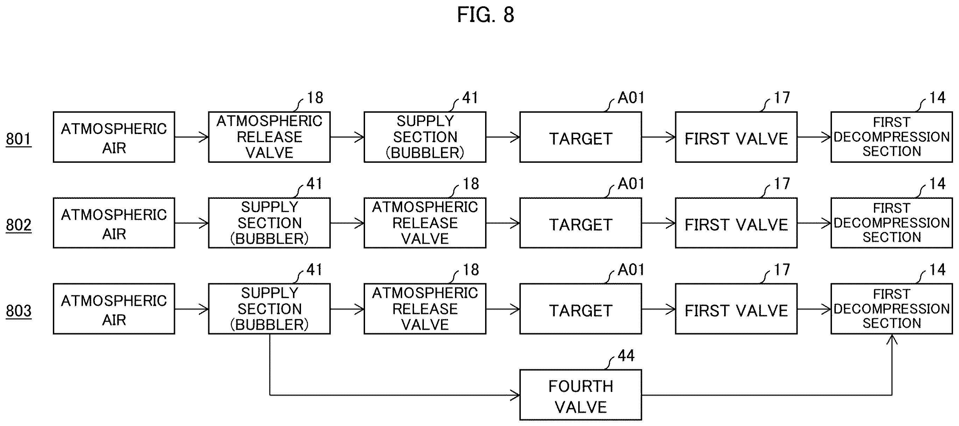

Fig. 8 is a view schematically illustrating flow of gas in a sterilization device. The configuration indicated by reference numeral 801 corresponds to the sterilization device 4. In the configuration indicated by reference numeral 801, the atmospheric release valve 18 is provided between the supply section 41 constituting a bubbler and the atmospheric air. In contrast, no valve is provided between the supply section 41 and the target A01. The first container 12 including the target A01 and the supply section 41 is a vacuum vessel. While the first decompression process is being carried out, the first valve 17 is open and the atmospheric release valve 18 is closed. Therefore, a space around the target A01 and a space around the peroxynitric acid solution in the supply section 41 are subjected to vacuuming. Thereafter, the control section 19 closes the first valve 17 and opens the atmospheric release valve 18. This allows air to be introduced into the peroxynitric acid solution in the supply section 41, thus causing bubbling. The peroxynitric acid gas generated in the supply section 41 is supplied to a space around the target A01 that has been subjected to pressure reduction in advance.

In the configuration indicated by reference numeral 802, the atmospheric release valve 18 is provided between the supply section 41 constituting a bubbler and the target A01. While the first decompression process is being carried out, the first valve 17 is open and the atmospheric release valve 18 is closed. Therefore, only a space around the target A01 is subjected to vacuuming. Thereafter, the control section 19 closes the first valve 17 and opens the atmospheric release valve 18. At this time, the air above the peroxynitric acid solution in the supply section 41 is at the atmospheric pressure. Therefore, the air originally remaining above the peroxynitric acid solution first flows to a space around the target A01, and then the peroxynitric acid gas generated by bubbling flows to the space around the target A01.

Therefore, the configuration indicated by reference numeral 801 can supply high-concentration peroxynitric acid gas to a space around the target A01 from an initial stage, compared to the configuration indicated by reference numeral 802.

In the configuration indicated by reference numeral 802, depending on the shape of the target A01, the air that contains no peroxynitric acid gas and that is supplied first may stagnate around (or inside) the target A01 and prevent the peroxynitric acid gas supplied later from coming into contact with the target A01. In order to sterilize microorganisms adhering to the surface of the target A01, it becomes necessary to secure a period for the air stagnating therearound and the peroxynitric acid gas supplied later to be sufficiently mixed by diffusion. This means that during the period required for the diffusion, the concentration of the peroxynitric acid gas with a short half-life decreases, resulting in failure to supply a sufficient concentration of the peroxynitric acid gas to the outermost surface of the target A01 to which the microorganisms adhere, and the time required for sterilization becomes longer.

In contrast, with the configuration indicated by reference numeral 801, since the air in the space above the peroxynitric acid solution in the supply section 41 has been subjected to vacuuming in advance, it is possible to prevent air from stagnating around the target A01. Therefore, the configuration indicated by reference numeral 801 (the configuration of the sterilization device 4) is particularly excellent in sterilization performance.

Note that in order to prevent this problem, the timing for opening/closing each valve may be changed in the configuration indicated by reference numeral 802. For example, while the first decompression process is being carried out, the first valve 17 is open and the atmospheric release valve 18 is closed. Thereafter, the control section 19 opens the atmospheric release valve 18 with the first valve 17 maintained open. This allows the air above the peroxynitric acid solution in the supply section 41 to be subjected to vacuuming, and the air supplied to a space around the target AO1 is discharged by the first decompression section 14. Thereafter, the control section 19 closes the first valve 17. This allows high-concentration peroxynitric acid gas to be supplied to the space around the target A01.

The configuration indicated by reference numeral 803 further includes a fourth valve 44 in addition to the configuration indicated by reference numeral 802. The fourth valve 44 connects the first decompression section 14 and a space above the peroxynitric acid solution in the supply section 41. While the first decompression process is being carried out, the first valve 17 is open, the atmospheric release valve 18 is closed, and the fourth valve 44 is closed. Thereafter, the first valve 17 is closed, and the fourth valve 44 is opened. This allows air above the peroxynitric acid solution in the supply section 41 to be subjected to vacuuming in advance. Thereafter, the control section 19 opens the atmospheric release valve 18 and closes the fourth valve 44. This allows air to be introduced to the peroxynitric acid solution in the supply section 41, and the peroxynitric acid gas generated by the bubbling flows to a space around the target A01. Also with the configuration indicated by reference numeral 803, it is possible to prevent air from stagnating around the target A01.

Note that in the configurations indicated by reference numerals 801, 802, and 803, a plurality of atmospheric release valves 18 may be provided in parallel instead of the single atmospheric release valve 18. If a large amount of air is suddenly supplied to the supply section 41 serving as a bubbler immediately after the start of the atmospheric release process, there is a possibility that the peroxynitric acid solution may scatter due to a water hammer phenomenon. Therefore, by opening the plurality of atmospheric release valves 18 in stages, it is possible to mitigate the impact and suppress scattering of the peroxynitric acid solution. The control section 19 may open the plurality of atmospheric release valves 18 in stages according to the pressure inside the first container 12 or time.

Further, some of the plurality of atmospheric release valves 18 provided in parallel may be open while the first decompression process is being carried out. In this case, a small amount of peroxynitric acid gas is supplied from the supply section 41 to the space around the target A01 during vacuuming. Under low pressure, the half-life of the peroxynitric acid gas becomes longer. Therefore, it is useful to continue supplying the peroxynitric acid gas also during the period of the first decompression process. Further, with the configuration indicated by reference numeral 802, by doing so, it is possible to, when the atmospheric release process is carried out, prevent the air (with a low concentration of peroxynitric acid gas) that has accumulated above the peroxynitric acid solution from being supplied to the space around the target A01.

The inventors conducted the following experiment in order to confirm the effect of the sterilization method and the sterilization device 4 in accordance with the embodiment.

A syringe was connected to a tube made of fluorine resin. In the syringe, a biological indicator (manufactured by MesaLabs, Inc., model number: HMV-091) was disposed. Furthermore, a member in which the whole tube and syringe were placed in a non-woven fabric bag (sterilization bag) was used as a process challenge device (PCD). The tube had a length of 5 m, an inner diameter of 1.0 mm, and an outer diameter of 3.0 mm. Compared to similar PCDs, the tube is longer, the number of non-woven fabrics is larger, and the sterilization resistance is high. The capacity of the vacuum vessel accommodating the PCD was 48 L.

For the sterilization device 4, one cycle included a one-minute first decompression process (with the first valve 17 opened and the atmospheric release valve 18 closed) and a one-minute atmospheric release process (with the first valve 17 closed and the atmospheric release valve 18 opened). In the first decompression process, the pressure inside the vacuum vessel was reduced to 0.02 atm. In the atmospheric release process, air was introduced into the vacuum vessel until the pressure became 1.0 atm. Sterilization was achieved in all PCDs across a plurality of experiments (n=6, microbial survival probability of 10⁻⁶) by applying a fourcycle (eight-minute) process. As described above, the sterilization device 4 can sterilize even a deep part of an instrument having an elongated lumen, such as a catheter or an endoscope.

In a case where a biological indicator (manufactured by MesaLabs, Inc., model number: HMV-091) alone was placed in the vacuum vessel as a PCD, sterilization (n=6) was achieved by a one-cycle (two-minute) process.

A carrier (SUS disk) to which bacterial spores were applied was taken out from the non-woven fabric of a biological indicator (manufactured by MesaLabs, Inc., model number: HMV-091) and placed into a syringe to which a tube was connected. A resulting member was entirely covered with a sterilization bag and used as a PCD. In a case where this PCD was placed in the vacuum vessel, sterilization (n=6) was achieved by a two-cycle (four-minute) process.

The foregoing has been a description of embodiments of the present disclosure. Note, however, that the present disclosure is not limited to the embodiments above, but can be put into practice in various aspects within the spirit of the present invention. Various embodiments can also be created through suitable combinations of a plurality of components described in the aforementioned embodiment or variations. For example, some components may be omitted from all the components shown in the embodiment or the variations. Furthermore, components across the embodiment and the variations may be suitably combined. For ease of understanding, the drawings schematically show each component as a main element, and the thickness, length, number, distance, and the like of the illustrated components may be different from actual ones for convenience of drawing. Further, the materials, shapes, dimensions, and the like of the components shown in the above-described embodiment are merely examples and are not particularly limited, and various modifications thereto are possible to the extent that they do not substantially depart from the effects of the present disclosure.
(1) In the embodiment, the atmospheric release process 193 opens the inside of the first container 12 to the atmospheric air through the atmospheric release port 16C and the flow path 16B so as to increase the pressure inside the first container 12. However, this should not be construed as a limitation. The pressure inside the first container 12 can be increased from the reduced pressure condition to a pressure other than the atmospheric pressure. Specifically, the atmospheric release process 193 is carried out in order to create a fluidic flow inside the first container 12. Therefore, the pressure inside the first container 12 is not necessarily returned to the atmospheric pressure. Specifically, depending on the structure of the target A01, if outside air can be introduced into the first container 12 up to a certain pressure, it becomes possible to prevent a decrease in the half-life associated with a pressure increase.
   The gas introduced into the first container 12 from the outside in the atmospheric release process is not limited to air, and may be another gas, such as nitrogen gas. That is, the expression "atmospheric release" is not limited to a process of increasing the pressure inside the first container 12 to the atmospheric pressure and is not limited to a process of introducing air. The "atmospheric release" refers to a gas supply process of supplying gas into the first container 12 from the outside to increase the pressure inside the first container 12.
(2) The first time T01, the second time T02, and the third time T03 may not be the examples described in the embodiment. Further, the number of the cycles of the first decompression process 191, the standstill process 192, and the atmospheric release process 193 may not be three.
(3) Nitrous acid gas may be fed to the first container 12. This causes recycling in the first container 12, and the stability of the peroxynitric acid gas under a reduced pressure condition is improved. Further, peroxynitric acid gas synthesized by using active species generated by plasma in combination may be used.
(4) In the embodiment, by the first decompression process 191 and the atmospheric release process 193, the first container 12 which is under the reduced pressure condition and is filled with the peroxynitric acid gas is rapidly opened to the atmospheric air with use of a mixed gas containing the peroxynitric acid gas. As a result, peroxynitric acid gas, which becomes unstable under the atmospheric pressure condition, is effectively supplied to a deep part of the target AO1. However, it is not necessary to carry out both the first decompression process 191 and the atmospheric release process 193. In the sterilization device 1, it is sufficient to carry out at least one of the first decompression process 191 and the atmospheric release process 193.
(5) Regarding the sterilization action by a chemical substance, it is generally known that its microbicidal activity is increased by the promotion of the chemical reaction with an increase in temperature. Therefore, by increasing the temperature inside the first container 12 with use of, for example, a heater before the start of the sterilization process by the sterilization device 1, it becomes possible to carry out a sterilization process in a short time.
(6) The peroxynitric acid gas has a half-life that is relatively long under a reduced pressure condition and is relatively short under the atmospheric pressure. Therefore, the sterilization activity of the peroxynitric acid gas is rapidly lost under the atmospheric pressure condition. In other words, the toxicity of the peroxynitric acid gas is also rapidly lost under the atmospheric pressure condition. In sterilization methods using ethylene oxide gas and hydrogen peroxide gas, due to their high toxicity, it is necessary to remove the sterilizing components remaining in the equipment by aeration for several hours or more after the sterilization process. However, in the sterilization method using peroxynitric acid gas, since the toxicity of the peroxynitric acid gas is rapidly lost under the atmospheric pressure condition, relatively long aeration is not required. Therefore, the sterilization device 1 in accordance with the present embodiment has very high industrial applicability.

### Industrial Applicability

The present invention can be applied to sterilization of, for example, medical instruments, cell culture isolators, and food containers, and is industrially applicable.

### Reference Signs List

1, 2, 3, 4: Sterilization device
11: Housing
12: First container
121: Opening
14: First decompression section
15: Supply section
151: Storage part
1511: Opening
1512: Absorbent body
16A, 16B, 181, 22A, 22B: Flow path
16C: Atmospheric release port
17: First valve
18: Atmospheric release valve (gas supply valve)
19: Control section
21: Second container
23: Second valve
24: Second decompression section
25: Third valve
31, 41: Supply section
43: Discharge pipe
433a, 433b: Discharge port

## Claims

1. A sterilization method comprising supplying a peroxynitric acid gas to a target placed under a reduced pressure condition in a first container.

2. The sterilization method according to claim 1, comprising:
a decompression step of reducing a pressure inside the first container; and
a gas supply step of supplying a gas into the first container to supply the peroxynitric acid gas to the target placed under a reduced pressure condition in the first container.

3. The sterilization method according to claim 2, wherein the decompression step and the gas supply step are repeated in sequence.

4. The sterilization method according to claim 3, wherein the decompression step is repeated at a period based on a half-life of the peroxynitric acid gas.

5. The sterilization method according to any one of claims 2 to 4, wherein
the first container is provided with a storage part storing a peroxynitric acid solution, and
in the gas supply step, the gas is supplied toward an opening of the storage part.

6. The sterilization method according to claim 5, wherein an absorbent body that absorbs the peroxynitric acid solution by capillary action is placed on a liquid surface of the peroxynitric acid solution in the storage part.

7. The sterilization method according to claim 1, wherein the peroxynitric acid gas obtained by bubbling a peroxynitric acid solution is supplied into the first container.

8. The sterilization method according to any one of claims 2 to 4, wherein
in the decompression step, a pressure in a space above a peroxynitric acid solution is reduced, and
in the gas supply step, the gas is supplied into the peroxynitric acid solution to carry out bubbling, and the peroxynitric acid gas thus obtained is supplied to the target.

9. A sterilization device comprising:
a first container capable of accommodating a target;
a first decompression section that reduces a pressure in the first container; and
a supply section that supplies a peroxynitric acid gas to the target that is placed under a reduced pressure condition by the first decompression section.

10. The sterilization device according to claim 9, comprising:
a first flow path connecting an inside of the first container and the first decompression section;
a first valve that opens and closes the first flow path;
a gas supply valve for increasing a pressure inside the first container; and
a control section,
the control section being configured to carry out
a first decompression process of opening the first valve, closing the gas supply valve, and reducing the pressure inside the first container with use of the first decompression section and
a gas supply process of opening the gas supply valve to supply a gas into the first container.

11. The sterilization device according to claim 10, further comprising:
a second container;
a second flow path connecting the inside of the first container and an inside of the second container;
a second valve that opens and closes the second flow path;
a second decompression section that reduces a pressure in the second container;
a third flow path connecting the inside of the second container and the second decompression section; and
a third valve that opens and closes the third flow path,
the control section being configured to further carry out
a second decompression process of opening the third valve and reducing a pressure inside the second container with use of the second decompression section and
a valve open process of opening the second valve while the first decompression process is being carried out.

12. The sterilization device according to claim 9, wherein
the supply section includes a storage part storing a peroxynitric acid solution,
the first decompression section reduces a pressure in a space above the peroxynitric acid solution in the storage part, and
the supply section supplies, in a state in which the pressure in the space above the peroxynitric acid solution is reduced, a gas into the peroxynitric acid solution to carry out bubbling and supplies the peroxynitric acid gas thus obtained to the target.

13. The sterilization device according to claim 12, wherein the supply section has a discharge port that discharges the gas in a circumferential direction in a position apart from a center of the storage part.
